# EUROPEAN PATENT SPECIFICATION

(11) **EP 1 541 633 B1**
(45) Date of publication and mention of the grant of the patent: **16.01.2013**
(21) Application number: 03791355.5
(22) Date of filing: 27.08.2003
(51) Int. Cl.: C08L 83/04

(54) **AQUEOUS SUSPENSION OF CROSSLINKED SILICONE PARTICLES, AQUEOUS EMULSION OF OIL CONTAINING CROSSLINKED SILICONE PARTICLES, AND COSMETIC INGREDIENTS**
WÄSSRIGE SUSPENSION VON TEILCHEN AUS VERNETZTEM SILIKON, WÄSSRIGE EMULSION VON ÖLHALTIGEN TEILCHEN AUS VERNETZTEM SILIKON UND KOSMETISCHE BESTANDTEILE
SUSPENSION AQUEUSE DE PARTICULES DE SILICONE RETICULEES, EMULSION AQUEUSE DE PARTICULES DE SILICONE RETICULEES CONTENANT DE L'HUILE ET PRODUITS COSMETIQUES

(30) Priority: 30.08.2002 JP 2002253540
(43) Date of publication of application: 15.06.2005
(73) Proprietor: Dow Corning Toray Co., Ltd., Tokyo, 100-0004 (JP)
(72) Inventor: MORITA, Y., Dow Corning Toray Silicone Co. Ltd, Ichihara-shi, Chiba 299-0108 (JP); KOBAYASHI, K., Dow Corning Toray Silicone Co. Ltd, Ichihara-shi, Chiba 299-0108 (JP); OZAKI, M., Dow Corning Toray Silicone Co. Ltd, Ichihara-shi, Chiba 299-0108 (JP)
(74) Representative: Gillard, Richard Edward
(86) International application number: PCT/JP2003/010888
(87) International publication number: WO 2004/020526

(56) References cited:
- EP-A2- 1 151 743
- WO-A-98/13011
- WO-A-99/32079
- WO-A1-02/072703
- WO-A1-02/088253
- WO-A2-02/094213
- JP-A- 2 243 612
- JP-A- 3 017 006
- JP-A- 4 296 398
- JP-A- 11 140 191
- JP-A- 11 217 324
- JP-A- 11 506 125
- JP-A- 56 036 546
- JP-A- 2003 286 147

## Description

### Technical Field

The present invention relates to aqueous suspensions of cross-linked silicone particles, aqueous emulsions of oil containing cross-linked silicone particles, and cosmetic raw materials. More specifically, it relates to aqueous suspensions of cross-linked silicone particles, aqueous emulsions of oil containing cross-linked silicone particles, and cosmetic raw materials of superior stability, low environmental impact and minimal effects on the human body.

### Background Art

Aqueous suspensions of cross-linked silicone particles composed of cross-linked silicone particles, surface active agents, and water, as well as aqueous emulsions of oil containing cross-linked silicone particles have been known in the past (see Japanese Unexamined Patent Application Publication No. Sho 63-309565, Japanese Unexamined Patent Application Publication No. Hei 11-140191, and Japanese Unexamined Patent Application Publication No. 2000-281903).

In addition, when added to water-base paints, such suspensions produce a delustered dry paint layer (see Japanese Unexamined Patent Application Publication No. Hei 5-9409), and it has been suggested that using them in water-base cosmetic produces improved sensory properties (see Japanese Unexamined Patent Application Publication No. Hei 10-139624 and Japanese Unexamined Patent Application Publication No. Hei 10-175816).

Nonionic surface active agents, anionic surface active agents, cationic surface active agents, and amphoteric surface active agents, or surface active agents made up of their mixtures are used in such aqueous suspensions and aqueous emulsions, with alkyl polyethers and other nonionic surface active agents being preferable due to the excellent dispersibility of the cross-linked silicone particles and oils containing cross-linked silicone particles obtained when such agents are used for cosmetic applications.

WO 99/32079 relates to shampoo compositions comprising microemulsified particles of a high viscosity silicone having a particle size of ≤ 0.15 microns, a surfactant, a deposition polymer which is the cationic derivative of guar gum and an aqueous carrier.

WO 98/13011 describes a conditioning shampoo composition for hair and/or skin comprising a stable microemulsion of high viscosity, slightly cross-linked silicone with a particle size of < 0.15 microns, a deposition polymer and a surfactant.

In recent years, it has been required that surface active agents of low environmental impact be selected for use in such aqueous suspensions and aqueous emulsions while at the same time ensuring their improved stability and dispersibility in mixtures. For instance, chemical substances of environmental concern such as alkyl polyethers bearing **C₁₂₋₁₅** alkyl groups are recognized as designated PRTR (Pollutant Release and Transfer Register) chemical substances whose release volumes etc. have been subject to monitoring. In addition, according to a Notification from the Head of the Pharmaceutical and Medical Safety Bureau of the Ministry of Health, Labour and Welfare entitled "On assuring the quality and safety of medicines etc. manufactured using raw materials of bovine origin" (December 12, 2000), restrictions are still in effect on the cosmetics use of surface active agents derived from animal fats using raw materials such as beef tallow etc. and especially animal fat-derived surface active agents with alkyl groups.

As a result of in-depth investigations aimed at eliminating the above-described problems, the present inventors arrived at the present invention by discovering that the above-described problems can be eliminated by selecting specific N-acyl-, N-hydrocarbon taurines and/or their salts as surface active agents for use in aqueous suspensions of cross-linked silicone particles and aqueous emulsions of oil containing cross-linked silicone particles.

Namely, it is an object of the present invention to provide aqueous suspensions of cross-linked silicone particles and aqueous emulsions of oil containing cross-linked silicone particles possessing superior stability, low environmental impact and minimal effects on the human body, as well as cosmetic raw materials utilizing said aqueous suspensions and aqueous emulsions.

### Disclosure of the Invention

The present invention relates to aqueous suspensions of cross-linked silicone particles comprising :
(A) cross-linked silicone particles with an average particle size of from 0.5 to 500 µm,
(B) N-acyl-, N-hydrocarbon taurines represented by the general formula (I) ; 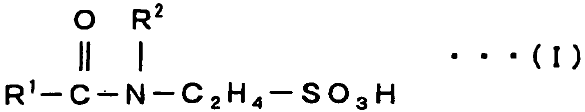 (where R¹ and R² stand for unsubstituted or substituted monovalent hydrocarbon groups) and/or their salts, and
(C) water,
and to cosmetic raw materials comprising said aqueous suspensions, as well as to aqueous emulsions of oils containing cross-linked silicone particles comprising :
(A) cross-linked silicone particles with an average particle size of from 0.5 to 500 µm,
(D) oil,
(B) N-acyl-, N-hydrocarbon taurines represented by the general formula (I) ; 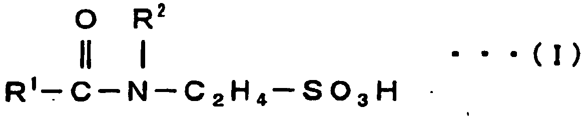 (where R¹ and R² stand for unsubstituted or substituted monovalent hydrocarbon groups) and/or their salts, and
(C) water,
   with component (A) contained in droplets of component (D) dispersed in water,
   and to cosmetic raw materials comprising said aqueous emulsions.

### Detailed Description of the Invention

First of all, explanations are provided regarding the inventive aqueous suspensions of cross-linked silicone particles.

The cross-linked silicone particles (A) constitute the principal component of the inventive suspensions, their average particle size being from 0.1 to 500 µm, preferably from 0.1 to 100 µm, even more preferably from 0.1 to 50 µm, and especially preferably from 0.5 to 50 µm. This is due to the fact that cross-linked silicone particles whose average particle size is below the lower limits of the above-mentioned ranges are difficult to prepare, and, on the other hand, when the size exceeds the upper limits of the above-mentioned ranges, the dispersibility of the suspensions in cosmetic, paint, etc. tends to decrease. The shape of component (A) may be, for instance, spherical, oblate, or irregular, with the spherical shape being more preferable due to the particularly high dispersibility of the resultant suspensions in cosmetic, paint, etc. In addition, the consistency of component (A) may be, for instance, similar to that of hard rubber, soft rubber, or gel.

More specifically, hydrosilylation reaction-crosslinkable silicone compositions, condensation reaction-crosslinkable silicone compositions, organic peroxide-crosslinkable silicone compositions, and UV-crosslinkable silicone compositions are suggested as the cross-linkable silicone compositions used in the preparation of component (A). Among them, hydrosilylation reaction-crosslinkable silicone compositions and condensation reaction-crosslinkable silicone compositions are preferable.

Compositions containing organopolysiloxanes having at least two alkenyl groups per molecule, organohydrogenpolysiloxanes having at least two silicon-bonded hydrogen atoms per molecule, and platinum catalysts as their main ingredients are suggested as examples of the hydrosilylation reaction-crosslinkable silicone compositions.

Compositions, whose main ingredients include organopolysiloxanes having, per molecule, at least two hydrolysable groups such as aminoxy, acetoxy, oxime, alkoxy, or hydroxyl groups bonded to silicon atoms, silane cross-linking agents having, per molecule, at least three hydrolysable groups such as aminoxy, acetoxy, oxime, and alkoxy bonded to silicon atoms, and condensation reaction catalysts, such as organotin or organotitanium compounds, are suggested as examples of the condensation reaction-crosslinkable silicone compositions. Preferably, these are silicone compositions crosslinkable by the dealcoholation condensation reaction, whose main ingredients include organopolysiloxanes having, per molecule, at least two alkoxy groups or hydroxyl groups bonded to silicon atoms, silane crosslinking agents having, per molecule, at least three alkoxy groups bonded to silicon atoms, and condensation reaction catalysts such as organotin compounds, organotitanium compounds, etc.

In addition, component (A) may contain non-crosslinkable oils. There are no particular limitations on the oils, with suggested oils including silicone oils and organic oils. The molecular structure of the non-crosslinkable silicone oils could be, for instance, linear, partially branched linear, cyclic, or branched, with linear and cyclic structures being particularly preferable. For instance, dimethylpolysiloxanes having both ends of the molecular chain blocked by trimethylsiloxy groups, cyclic dimethylsiloxanes, silicone oils obtained by substituting ethyl, propyl, butyl, and other alkyl groups, phenyl groups, or 3,3,3-trifluoropropyl groups for some of the methyl groups of the above-mentioned silicone oils, as well as mixtures of the above-mentioned silicone oils are suggested as such silicone oils. Suggested examples of the non-crosslinkable organic oils include, for instance, liquid paraffin, isoparaffin, hexyl laurate, isopropyl myristate, myristyl myristate, cetyl myristate, 2-octyldodecyl myristate, isopropyl palmitate, 2-ethylhexyl palmitate, butyl stearate, decyl oleate, 2-octyldodecyl oleate, myristyl lactate, cetyl lactate, lanolin acetate, stearyl alcohol, cetostearyl alcohol, oleyl alcohol, avocado oil, almond oil, olive oil, cacao oil, jojoba oil, sesame oil, safflower oil, soybean oil, tsubaki oil, squalane, persic oil, castor oil, cottonseed oil, coconut oil, polypropylene glycol monooleate, neopentyl glycol-2-ethylhexanoate, isostearic acid triglyceridess, coconut oil fatty acid triglycerides, polyoxyethylene lauryl ether, and polyoxypropylene cetyl ether. Of the above, silicone oils are preferable due to their superior affinity to the cross-linked silicone particles. Their viscosity at 25 °C is preferably not more than 100,000 mm²/s, more preferably, not more than 50,000 mm²/s, and especially preferably, not more than 10,000 mm²/s. Although there are no limitations on the content of the non-crosslinkable oils used in component (A), their content is preferably not more than 50 % by weight, more preferably, not more than 30 % by weight, and even more preferably, not more than 10 % by weight. This is due to the fact that when their content exceeds 50 % by weight, component (A) cannot hold any more of the non-crosslinkable oils and there is chance that this may lead to the formation of the aqueous emulsions of oil containing cross-linked silicone particles described below. Cross-linked silicone particles containing such non-crosslinkable oils can be prepared by combining the aforementioned cross-linkable silicone compositions with non-crosslinkable oils and using organopolysiloxanes containing low molecular weight siloxane oligomers as a raw material for the cross-linkable silicone compositions.

Although there are no limitations on the content of component (A) in the suspensions of the present invention, the content is preferably from 25 to 80 % by weight, and even more preferably, from 40 to 80 % by weight. This is due to the fact that when the content of component (A) is below the lower limits of the above-mentioned ranges, the scope of uses of the suspensions may be limited, and, on the other hand, when the content exceeds the upper limits of the above-mentioned ranges, its properties during handling and use deteriorate and its dispersibility in cosmetic, paint, etc. tends to decrease.

(B) N-acyl-, N-hydrocarbon taurines and/or their salts constitute a characteristic component of the present invention used as a surface active agent. In addition, not only does the component improve the stability of the suspensions, but it also improves their dispersibility and stability in combinations with other components; besides, it is friendly to the environment and to the human body. Component (B) consists of N-acyl-, N-hydrocarbon taurines represented by the general formula (I) ; 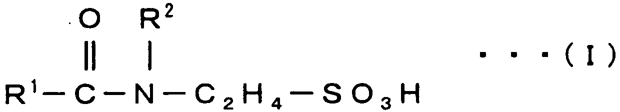 and/or their salts. In the formula above, R¹ and R² are unsubstituted or substituted monovalent hydrocarbon groups specifically exemplified by methyl, ethyl, propyl, butyl, pentyl, hexyl, heptyl, octyl, decyl, dodecyl, myristyl, palmityl, stearyl, and other alkyl groups; vinyl, allyl, hexenyl, oleyl, and other alkenyl groups; cyclopentyl, cyclohexyl, and other cycloalkyl groups; phenyl, tolyl, naphthyl, and other aryl groups. R¹ and R² may be identical or different, with alkyl, alkenyl, or cycloalkyl groups being preferable, and alkenyl or alkyl groups with from 5 through 30 carbon atoms even more preferable in case of R¹. R² is preferably an alkyl group, usually methyl. The term N-acyl taurines generally refers to compounds, in which hydrogen atoms are bonded to nitrogen atoms according to the formula above (compounds, in which R² stands for a hydrogen atom), with the N-acyl-, N-hydrocarbon taurines of the present invention characterized by nitrogen-bonded acyl groups and monovalent hydrocarbon groups. In addition, the salts of such N-acyl-, and N-hydrocarbon taurines are formed by neutralizing them with alkaline substances such as sodium hydroxide, potassium hydroxide and other alkali metal hydroxides; sodium carbonate and other weak acid salts of alkali metals; ammonia, triethanolamine, ammonium hydroxide, and other water-soluble amines; basic amino acids; taurine salts. The taurine salts used as neutralizing agents are represented by the general formula ; 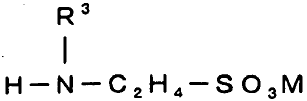 (where R³ stands for a hydrogen atom or an alkyl group, and M is an alkali metal) and are specifically exemplified by compounds represented by the following formulae. 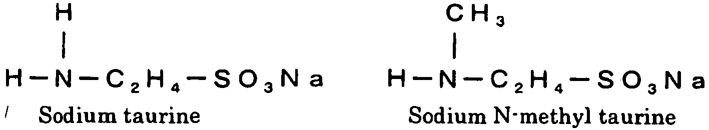 Component (B) is exemplified by sodium N-lauroyl methyl taurine, sodium N-myristoyl methyl taurine, sodium N-oleoyl methyl taurine, sodium N-stearoyl methyl taurine, sodium N-coconut fatty acid methyl taurine, potassium N-coconut fatty acid methyl taurine, magnesium N-coconut fatty acid methyl taurine, sodium N-palmitoyl methyl taurine, potassium N-stearoyl methyl taurine, potassium N-cetyloyl methyl taurine and non-neutralized products thereof. The above-mentioned compounds can be used singly or as mixtures combining several components.

Although there are no limitations on the content of component (B) in the suspension of the present invention, the content is preferably from 0.001 to 20 % by weight, and especially preferably, from 0.01 to 10 % by weight. This is due to the fact that when the content of component (B) is below the lower limits of the above-mentioned ranges, the dispersibility of the suspensions in cosmetic, paint, etc. tends to deteriorate, and, on the other hand, when the content exceeds the upper limits of the above-mentioned ranges, the scope of their uses may be limited.

Component (C), i.e. water, which serves as the dispersion medium for component (A), may be, for instance, demineralized water or ion exchange water. Although there are no limitations on the content of component (C) in the suspension of the present invention, the content is preferably from 5 to 75 % by weight, and especially preferably, from 10 to 60 % by weight.

Furthermore, phenoxyethanol, ethanol, i-propanol, t-butanol, ethylene glycol, propylene glycol, diethylene glycol, and other alcohols; carboxyvinyl polymers, sodium carboxymethyl cellulose, and other water-soluble polymers; amino-containing silicones, polyether-containing silicones, and other organosilicon compounds, and, in addition, antiseptic agents, antimicrobial agents, anti-fungal agents, anticorrosive agents, fragrances, and pigments can be combined with the suspensions of the present invention as optional components. In addition, so long as the purpose of the present invention is not impaired, in order to improve the stability of the suspensions in mixtures with other components, they may be combined with various surface active agents other than non-ionic surface active agents, such as anionic surface active agents, cationic surface active agents, and amphoteric surface active agents. The components may be used singly or as combinations of several components. In addition, there are no particular limitations on the order, in which they are to be combined.

There are no limitations on the methods used to prepare the suspensions of the present invention, with suggested techniques including, for instance, adding and dispersing the above-mentioned cross-linkable silicone compositions in an aqueous solution made up of component (B), component (C), and other optional components, and then cross-linking said compositions, or adding and dispersing silicone compositions obtained by removing crosslinking catalysts from the above-mentioned cross-linkable compositions to an aqueous solution made up of component (B), component (C), and other optional components and then cross-linking the above-mentioned compositions by adding the above-mentioned catalysts. In the latter technique, it is preferable to add the cross-linking catalyst after first dispersing it in an aqueous solution of component (B).

Next, detailed explanations are provided regarding the inventive aqueous emulsions of oil containing cross-linked silicone particles.

Cross-linked silicone particles (A) constitute the principal component of the inventive emulsion, with their average particle size being from 0.1 to 500 µm, preferably from 0.1 to 100 µm, even more preferably from 0.1 to 50 µm, and especially preferably from 0.5 to 50 µm. This is due to the fact that cross-linked silicone particles whose average particle size is below the lower limits of the above-mentioned ranges are difficult to prepare, and, on the other hand, when the average particle size exceeds the upper limits of the above-mentioned ranges, the stability of the emulsions tends to decrease. The shape of component (A) may be, for instance, spherical, oblate, or irregular, with the spherical shape being more preferable among them. In addition, the consistency of component (A) may be, for instance, similar to that of hard rubber, soft rubber, or gel.

Hydrosilylation reaction-crosslinkable silicone compositions, condensation reaction-crosslinkable silicone compositions, organic peroxide-crosslinkable silicone compositions, and UV-crosslinkable silicone compositions are suggested as examples of the cross-linkable silicone compositions used in the preparation of component (A) for use in the emulsions of the present invention. Among them, hydrosilylation reaction-crosslinkable silicone compositions and condensation reaction-crosslinkable silicone compositions, exemplified by the same compositions as those described above, are preferable.

(D) oil is also a major ingredient of the emulsions of the present invention and while there are no particular limitations on this component, suggested oils include silicone oils and organic oils, exemplified by the same non-crosslinkable oils as those mentioned above. Of these, silicone oils are preferable due to their superior affinity to the cross-linked silicone particles. In addition, their viscosity at 25 °C is preferably not more than 100,000 mm²/s, more preferably, not more than 50,000 mm²/s, and especially preferably, not more than 10,000 mm²/s. Component (D) is dispersed in water in the form of droplets containing (A) cross-linked silicone particles. Consequently, the average size of the droplets of component (D) is larger than that of component (A); specifically, it is, preferably, between 0.5 and 1,000 µm, and even more preferably, between 1 and 1,000 µm.

There are no limitations on the content of component (D), and while it is dependent on the oil absorbency of component (A), the weight of component (D) is preferably greater than 50 % by weight, and especially preferably, is not less than 60 % by weight relative to the combined weight of component (A) and component (D). This is due to the concern that if the content of component (D) is 50 % by weight or lower, it may end up contained in component (A), and it may be impossible to introduce component (A) in component (D).

While there are no limitations on the content of component (D) and component (A) in the emulsions of the present invention, i.e. there are no limitations on the content of the oil droplets containing cross-linked silicone particles, the content is preferably in the range of from 25 to 90 % by weight, and even more preferably, from 40 to 80 % by weight. This is due to the fact that when the content is below the lower limits of the above-mentioned ranges, the scope of uses of the emulsions may be limited, and, on the other hand, when it exceeds the upper limits of the above-mentioned ranges, their properties during handling and use deteriorate and their dispersibility in cosmetic, paint, etc. tends to decrease.

(B) N-acyl-, N-hydrocarbon taurines and/or their salts are a characteristic component of the present invention used a surface active agent. In addition, not only does this component improve the stability of the inventive emulsion, but it also improves its dispersibility and stability when combined with other components; besides, it is friendly to the environment and the human body. The same specific compounds as those mentioned above are suggested.

Although there are no limitations on the content of component (B) in the emulsions of the present invention, the content is preferably from 0.001 to 20 % by weight, and especially preferably, from 0.01 to 10 % by weight. This is due to the fact that when the content of component (B) is below the lower limits of the above-mentioned ranges, the dispersibility of the emulsions in cosmetic, paint, etc. tends to deteriorate, and, on the other hand, when the content exceeds the upper limits of the above-mentioned ranges, the scope of their uses may be limited.

Component (C), i.e. water, which serves as the dispersion medium, may be, for instance, demineralized water or ion exchange water. Although there are no limitations on the content of component (C) in the emulsions of the present invention, the content is preferably from 5 to 75 % by weight, and, especially preferably, from 10 to 60 % by weight.

Furthermore, phenoxyethanol, ethanol, i-propanol, t-butanol, ethylene glycol, propylene glycol, diethylene glycol, and other alcohols; carboxyvinyl polymers, sodium carboxymethyl cellulose, and other water-soluble polymers; amino-containing silicones, polyether-containing silicones, and other organosilicon compounds, and, in addition, antiseptic agents, antimicrobial agents, anti-fungal agents, anticorrosive agents, fragrances, and pigments can be combined with the emulsions of the present invention as optional components.

In addition, as long as the purpose of the present invention is not impaired, in order to improve the stability of the emulsions in mixtures with other components, it may be combined with various surface active agents such as anionic surface active agents, cationic surface active agents, amphoteric surface active agents, and nonionic surface active agents. The components may be used singly or as combinations of several components. In addition, there are no particular limitations on the order, in which they are to be combined.

There are no limitations on the methods used in the preparation of the emulsions of the present invention, with suggested techniques including, for instance, adding and dispersing cross-linkable silicone compositions containing component (D) in an aqueous solution made up of component (B), component (C), and other optional components, and then cross-linking the above-mentioned compositions; or adding and dispersing silicone compositions obtained by removing crosslinking catalysts from the above-mentioned cross-linkable compositions containing component (D) in an aqueous solution made up of component (B), component (C), and other optional components and then cross-linking the above-mentioned compositions by adding the above-mentioned catalysts. In the latter technique, it is preferable to add the cross-linking catalyst after first dispersing it in an aqueous solution of component (B).

The inventive aqueous suspensions and aqueous emulsions described above are characterized by superior stability as well as by low environmental impact and minimal effects on the human body. Furthermore, they are useful as raw materials for cosmetic and raw materials for paint because of their excellent stability in mixtures and dispersibility in cosmetic, paint, etc. and their ability to provide the full effects of adding cross-linked silicone particles when they are mixed with cosmetic or paint. In particular, they are extremely suitable as silicone raw materials mixed with cosmetic because they possess superior cosmetic functionality, such as wetness and smoothness, along with superior compatibility with the human body.

Next, explanations are provided regarding the cosmetic raw materials of the present invention.

The cosmetic raw materials of the present invention comprise the above-described aqueous suspensions or aqueous emulsions, and other silicone emulsion-based components known as additives for cosmetic raw materials can be combined with them so long as the object of the present invention is not impaired. Anticorrosive agents, anti-fungal agents, antiseptic agents, pH adjusting agents, nonionic surface active agents, and anionic surface active agents other than component (B), etc. are suggested as such additives. Such components can be used singly or as a combination of several components. There are no particular limitations on the order, in which they are to be combined. The content of the above-described aqueous suspensions and aqueous emulsions in the cosmetic raw materials of the present invention is preferably from 30 to 100 % by weight, and, even more preferably, from 50 to 100 % by weight.

Diethanolamine N-acyl-L-glutamate, triethanolamine N-acyl-L-glutamate, sodium N-acyl-L-glutamate, sodium alkane sulfonate, ammonium alkyl (12, 14, 16) sulfate, triethanolamine (1) alkyl (11, 13, 15) sulfate, triethanolamine (2) alkyl (11, 13, 15) sulfate, triethanolamine alkyl (12 through 14) sulfate, triethanolamine alkyl sulfate solution, sodium alkyl (12, 13) sulfate, sodium alkyl sulfate solution, sodium isethionate, sodium lactoisostearate, disodium undecylenoyl amidoethyl sulfosuccinate, triethanolamine sulfooleate, sodium sulfooleate, disodium oleamido sulfosuccinate, potassium oleate, sodium oleate, morpholine oleate, oleyl sarcosine, sodium methyl oleoyl taurine, potassium-containing soap base, potassium soap base solution, potassium soap, carboxylated polyoxyethylene tridodecyl ether, sodium polyoxyethylene tridodecyl ether carboxylate (3 E.O.), triethanolamine N-hydrogenated tallow acyl-L-glutamate, sodium N-hydrogenated tallow acyl-L-glutamate, sodium hydrogenated coconut oil fatty acid glyceryl sulfate, sodium diundecylenoyl amidoethyl sulfosuccinate, sodium stearyl sulfate, potassium stearate, triethanolamine stearate, sodium stearate, sodium N-stearoyl-L-glutamate, disodium stearoyl-L-glutamate, sodium dioctyl sulfosuccinate, sodium dioctyl sulfosuccinate solution, disodium polyoxyethylene monooleylamide sulfosuccinate solution (2 E.O.), disodium polyoxyethylene lauroyl ethanolamide sulfosuccinate solution (5 E.O.), disodium lauryl sulfosuccinate, diethanolamide cetyl sulfate, sodium cetyl sulfate, soap base, sodium cetostearyl sulfate, triethanolamine tridecyl sulfate, potassium palmitate, sodium palmitate, sodium methyl palmitoyl taurine, sodium ricinoleate solution (30%), polyoxyethylene alkyl ether sulfate solution (3 E.O.), diethanolamine polyoxyethylene alkyl (12, 13) ether sulfate solution (3 E.O.), triethanolamine polyoxyethylene alkyl ether sulfate solution (3 E.O.), triethanolamine polyoxyethylene alkyl (11, 13, 15) ether sulfate (1 E.O.), triethanolamine polyoxyethylene alkyl (12, 13) ether sulfate (3 E.O.), sodium polyoxyethylene alkyl ether sulfate solution (3 E.O.), sodium polyoxyethylene alkyl (11, 13, 15) ether sulfate (1 E.O.), sodium polyoxyethylene alkyl (11 through 15) ether sulfate (3 E.O.), sodium polyoxyethylene alkyl (12, 13) ether sulfate (3 E.O.), sodium polyoxyethylene alkyl (12 through 14) ether sulfate (3 E.O.), sodium polyoxyethylene alkyl (12 through 15) ether sulfate (3 E.O.), disodium polyoxyethylene alkyl (12 through 14) sulfosuccinate (7 E.O.), sodium polyoxyethylene undecyl ether sulfate, sodium polyoxyethylene octyl phenyl ether sulfate solution, ammonium polyoxyethylene oleyl ether sulfate, disodium polyoxyethylene lauryl sulfosuccinate, sodium polyoxyethylene nonyl phenyl ether sulfate, sodium polyoxyethylene pentadecyl ether sulfate, triethanolamine polyoxyethylene myristyl ether sulfate, sodium polyoxyethylene myristyl ether sulfate, sodium polyoxyethylene myristyl ether sulfate (3 E.O.), sodium polyoxyethylene lauryl ether acetate (16 E.O.) solution, ammonium polyoxyethylene lauryl ether sulfate (2 E.O.), triethanolamine polyoxyethylene lauryl ether sulfate, sodium polyoxyethylene lauryl ether sulfate, diethanolamine myristyl sulfate, sodium myristyl sulfate, potassium myristate, sodium N-myristoyl-L-glutamate, sodium myristoyl methylaminoacetate, sodium myristoyl methyl-β-alanine solution, sodium myristoyl methyl taurine, medicinal soap, triethanolamine-magnesium coco-alkyl sulfate, triethanolamine N-coco acyl-L-glutamate, sodium N-coco acyl-L-glutamate, sodium coco-ethyl ester sulfonate, potassium cocoate, potassium cocoate solution, sodium N-coco hydrogenated tallow acyl-L-glutamate, sarcosine cocoate, triethanolamine sarcosine cocoate, sodium sarcosine cocoate, triethanolamine cocoate, triethanolamine cocoate solution, sodium cocoate, sodium methyl alanine cocoate, sodium methyl alanine cocoate solution, potassium methyl taurine cocoate, sodium methyl taurine cocoate, sodium undecylaminodipropionate, sodium laurylaminodipropionate solution (30%), sodium lauryl sulfoacetate, sodium lauryl benzenesulfonate, laurylsulfuric acid, ammonium laurylsulfonate, potassium laurylsulfonate, diethanolamine laurylsulfonate, triethanolamine laurylsulfonate, sodium laurylsulfonate, magnesium laurylsulfonate, monoethanolamine lauryl sulfonate, potassium laurate, triethanolamine laurate, triethanolamine laurate solution, sodium laurate, triethanolamine myristate-laurate, triethanolamine lauroyl-L-glutamate, sodium N-lauroyl-L-glutamate, lauroyl sarcosine, potassium lauroyl sarcosine, triethanolamine lauroyl sarcosine solution, sodium lauroyl sarcosine, sodium lauroyl methyl-β-alanine solution, sodium lauroyl methyl taurine, and sodium lauroyl methyl taurine solution are suggested as specific examples of the anionic surface active agents.

Ethylene glycol fatty acid ethyl esters, polyethylene glycol fatty acid esters, propylene glycol fatty acid esters, polypropylene glycol fatty acid esters, glycol fatty acid esters, trimethylolpropane fatty acid esters, pentaerythritol fatty acid esters, glucoside derivatives, glycerol alkyl ether fatty acid esters, trimethylolpropane oxyethylene alkyl ethers, fatty acid amides, alkylolamides, alkylamine oxides, lanolin and its derivatives, castor oil derivatives, hydrogenated castor oil derivatives, sterols and their derivatives, polyoxyethylene alkyl ethers, polyoxyethylene alkyl allyl ethers, polyoxyethylene alkyl amines, polyoxyethylene fatty acid amides, polyoxyethylene alkylolamides, polyoxyethylene diethanolamine fatty acid esters, polyoxyethylene trimethylolpropane fatty acid esters, polyoxyethylene alkyl ether fatty acid esters, polyoxyethylene polyoxypropylene glycols, polyoxyethylene polyoxypropylene alkyl ethers, polyoxyethylene polyoxypropylene polyhydric alcohol ethers, glycerol fatty acid esters, polyglycerol fatty acid esters, polyoxyethylene glycerol fatty acid esters, sorbitan fatty acid esters, polyoxyethylene sorbitan fatty acid esters, and sucrose fatty acid esters are suggested as specific examples of the nonionic surface active agents.

Hydrochloric acid, sulfuric acid, phosphoric acid, diammonium hydrogen phosphate, disodium hydrogen phosphate, dipotassium hydrogen phosphate, ammonium dihydrogen phosphate, sodium dihydrogen phosphate, potassium dihydrogen phosphate, trisodium phosphate, tripotassium phosphate, acetic acid, ammonium acetate, sodium acetate, potassium acetate, citric acid, sodium citrate, ammonium citrate, sodium carbonate, potassium carbonate, ammonium carbonate, sodium hydrogen carbonate, ammonium hydrogen carbonate, sodium hydroxide, potassium hydroxide, ammonia, and triethanolamine are suggested as specific examples of the pH-adjusting agents.

Benzoic acid, aluminum benzoate, sodium benzoate, isopropyl methyl phenol, ethyl hexanediol, lysozyme chloride, chlorhexidine chloride, octyl phenoxyethanol, ortho-phenyl phenol, sodium perborate, photosensitive material No. 101, photosensitive material No. 201, photosensitive material No. 301, photosensitive material No. 301, photosensitive material No. 401, chlorhexidine gluconate solution, cresol, chloramine-T, chlorxylenol, chlorcresol, chlorphenesin, chlorhexidine, chlorobutanol, resorcin acetate, salicylic acid, sodium salicylate, domiphen bromide, zinc pyrithione, zinc pyrithione solution, sorbic acid, potassium sorbate, thiantol, thioxolone, thimol, thiram, dehydroacetic acid, sodium dehydroacetate, trichlorocarbanilide, trichlorohydroxydiphenyl ether, isobutyl para-oxybenzoate, isopropyl para-oxybenzoate, ethyl para-oxybenzoate, butyl para-oxybenzoate, propyl para-oxybenzoate, benzyl para-oxybenzoate, methyl para-oxybenzoate, sodium methyl para-oxybenzoate, parachlorphenol, sodium para-phenolsulfonate (dihydrate), halocarban, phenoxyethanol, phenol, hexachlorophane, mononitroguaiacol, sodium mononitroguaiacol, para-dimethylaminostyryl heptyl methyl triazolinium, lauryl trimethylammonium trichlorophenoxide, oxyquinoline sulfate, oxyquinoline phosphate, and resorcin are suggested as specific examples of the antiseptic agents, anti-fungal agents, and anticorrosive agents.

Skincare cosmetics possessing excellent affinity to the skin and capable of imparting superior wetness and smoothness are obtained by combining the above-described cosmetics of the present invention with the following various raw materials.

The various raw materials used in skincare cosmetics are exemplified by the above-mentioned anionic surface active agents, nonionic surface active agents, pH-adjusting agents, antiseptic agents, anti-fungal agents, anticorrosive agents, etc., as well as by avocado oil, almond oil, olive oil, cacao butter, sesame oil, wheat germ oil, safflower oil, shea butter, turtle oil, tung oil, persic oil, castor oil, grape seed oil, macadamia nut oil, mink oil, egg yolk oil, Japan wax, coconut oil, rosehip oil, hydrogenated oils, and other oils and fats; orange roughy oil, carnauba wax, candelilla wax, spermaceti wax, jojoba oil, montan wax, beeswax, lanolin, and other waxes; liquid paraffin, Vaseline, paraffin, cerisin, microcrystalline wax, squalane, and other hydrocarbons; lauric acid, myristic acid, palmitic acid, stearic acid, oleic acid, behenic acid, undecylenic acid, oxystearic acid, linolic acid, lanolic acid, synthetic fatty acids, and other higher fatty acids; ethyl alcohol, isopropyl alcohol, lauryl alcohol, cetyl alcohol, cetostearyl alcohol, stearyl alcohol, oleyl alcohol, behenyl alcohol, lanolin alcohol, hydrogenated lanolin alcohol, hexyldecanol, octyldodecanol, isostearyl alcohol, and other alcohols; cholesterol, dihydrocholesterol, phytosterol, and other sterols; ethyl linolate, isopropyl myristate, lanolin fatty acid isopropyl ester, hexyl laurate, myristyl myristate, cetyl myristate, octyldodecyl myristate, decyl oleate, octyldodecyl oleate, hexyldecyl dimethyloctanoate, cetyl isooctanoate, cetyl palmitate, glycerin trimyristate, glycerin tri(caprylate/caprate), propylene glycol dioleate, glycerin triisostearate, glycerin triisooctanoate, cetyl lactate, myristyl lactate, diisostearyl malate, and other fatty acid esters; glycerin, propylene glycol, 1,3-butylene glycol, polyethylene glycol, sodium d,1-pyrrolidonecarbonate, sodium lactate, sorbitol, sodium hyaluronate, and other humectants; cationic surface active agents; betaine-type, amino acid-type, imidazoline-type, lecitin-type and other amphoteric surface active agents; iron oxides and other colored pigments, zinc oxides, titanium oxides, zirconium oxides, and other white pigments, mica, talc, sericite, and other extender pigments; dimethylpolysiloxane, methylphenylpolysiloxane, octamethyltetracyclosiloxane, decamethylcyclopentasiloxane, polyether-modified silicone oils, amino-modified silicone oils, and other silicone oils; demineralized water; carrageenan, alginic acid, gum arabic, traganth, pectin, starches, xantham gum, polyvinyl alcohol, polyvinyl pyrrolidone, sodium polyacrylate, polyethylene glycol, and other thickeners; silicone-acrylic copolymer, silicone resins, acrylic polymers, and other coating-forming agents; and, furthermore, UV absorbers, anti-microbial agents, anti-inflammatory agents, antiperspirants, fragrances, antioxidants, and propellants. In addition, hand creams, skin creams, foundations, eye shadows, facial cleansers, and body shampoos are suggested as specific examples of the skincare cosmetics.

In addition, when the cosmetics of the present invention are used as hair care cosmetics, combining them with the above-mentioned anionic surface active agents, nonionic surface active agents, pH-adjusting agents, antiseptic agents, anti-fungal agents, and anticorrosive agents, as well as with film-forming agents, antifreeze agents, oils, emulsifying agents, lubricants, anti-dandruff agents, antioxidants, chelating agents, UV absorbers, fragrances, colorants, and various other raw materials makes it possible to obtain hair care cosmetics exhibiting excellent adhesion to hair and capable of imparting superior moisturizing properties and smoothness to it. Specifically, the film-forming agents are exemplified by polymers of (meth)acrylic radical-polymerizable monomers and copolymers with silicone compounds, poly(N-acylalkyleneimine), poly(N-methylpyrrolidone), non-functional silicone resins and silicone resins modified with fluorine-containing organic groups and amino groups. There are no particular limitations on the antifreeze agents, however, in general, suggested agents include ethanol, isopropyl alcohol, 1,3-butylene glycol, ethylene glycol, propylene glycol, and glycerin. Oils commonly used in cosmetics can be used as the oils. Microcrystalline wax, paraffin wax, spermaceti wax, beeswax, japan wax, sugarcane wax, and other waxes and their mixtures; liquid paraffin, α-olefin oligomers, squalane, squalene, and other hydrocarbon oils or their mixtures; cetanol, stearyl alcohol, isostearyl alcohol, hydrogenated castor oil-derived alcohols, behenyl alcohol, lanolin alcohol, and other linear or branched, saturated or unsaturated, unsubstituted or hydroxy-substituted higher alcohols or their mixtures, palmitic acid, myristic acid, oleic acid, stearic acid, hydroxystearic acid, isostearic acid, behenic acid, castor oil fatty acids, coconut oil fatty acids, tallow fatty acids, and other linear or branched, saturated or unsaturated, unsubstituted or hydroxy-substituted higher fatty acids or their mixtures, olive oil, coconut oil, rapeseed oil, palm oil, palm kernel oil, castor oil, hydrogenated castor oil, peanut oil, tallow, hydrogenated tallow, jojoba oil, hydrogenated jojoba oil, monostearic acid glyceride, monooleic acid glyceride, dipalmitic acid glyceride, trimyristic acid glyceride, oleyl oleate, isostearyl isostearate, palmityl behenate, isopropyl palmitate, stearyl acetate, dihydroxystearic acid ester, and other esters, linear, branched, or cyclic low molecular weight silicone oils, amino-modified silicone oils, fatty acid-modified silicone oils, alcohol-modified silicone oils, polyether-modified silicone oils, phosphoric acid (salt)-containing silicone oils, sulfuric acid (salt)-containing silicone oils, fluorine-modified alkyl-containing silicone oils, alkyl-modified silicone oils, epoxy-modified silicone oils and other silicone oils, high molecular weight silicones, raw rubber-like or thermoplastic silicone resins soluble in solvents and liquid at room temperature, or their mixtures, are suggested as their representative examples. The silicones are preferably latex emulsions, the emulsifying agents including, for instance, agents commonly used in the past, such as glycerin monostearate, sorbitan monopalmitate, polyoxyethylene cetyl ether, polyoxyethylene stearic acid ester and polyoxyethylene sorbitan monolaurate. Hexylene glycol, polyethylene glycol 600, sodium pyroglutamate, and glycerin are suggested as the humectants. The anti-dandruff agents are exemplified by sulfur, selenium sulfide, zinc pyridium-1-thiol-N-oxide, salicylic acid, 2,4,4'-trichloro-2'-hydroxydiphenyl ether, and 1-hydroxy-2-pyridone compounds. BHA, BHT, and γ-oryzanol are suggested as the antioxidants. The chelating agents are exemplified by ethylenediamine tetraacetate and ethane-1-hydroxy-1,1-diphosphonic acid and its salts. The UV absorbers are exemplified by benzophenone derivatives represented by 2-hydroxy-4-methoxybenzophenone, benzotriazole derivatives represented by 2-(2'-hydroxy-5'-methylphenyl)benzotriazole, cinnamic acid ester, etc. Furthermore, preferred compounds include glycerin, propylene glycol, dipropylene glycol, 1,3-butylene glycol and other polyhydric alcohols, monoalkyltrimethylammonium salts, dialkyldimethylammonium salts, and other quaternary ammonium salts, with suggested specific compounds including stearyltrimethylammonium chloride, behenyltrimethylammonium chloride, distearyldimethylammonium chloride, dibehenyldimethylammonium chloride, and other cationic surface active agents, or amphoteric surface active agents, squalane, lanolin, perfluoropolyether, cationic polymers, and other sensory property improvers, propylene glycol, glycerin, sorbitol, and other humectants, methylcellulose, carboxyvinyl polymers, hydroxyethyl cellulose, polyoxyethylene glycol distearate, ethanol, and other viscosity adjusters, pearlizers, fragrances, colorants, dyes, propellants, vitamins, hair nourishing formulas, hormones, and other drugs, triclosan, trichlorocarban, and other anti-microbial agents, potassium glycyrrhizinate, tocopherol acetate, and other anti-inflammatory agents, zinc pyrithion, octopyrox, and other anti-dandruff agents, methylparaben, butylparaben, and other antiseptic agents, atomizing agents, and other ingredients included in the Encyclopedia of Shampoo Ingredients (Micelle Press, 1985). In addition, shampoos, hair rinses, hair conditioners, hair treatments, setting lotions, blow-styling formulas, hair sprays, styling foams, styling gels, hair liquids, hair tonics, hair creams, hair growth formulas, hair nourishing agents, and hair-dyeing agents are specifically suggested as the hair care cosmetics.

### Examples

Below, the present invention is explained in detail by referring to practical examples. The viscosity values used in the application examples were obtained at 25 °C. The aqueous suspensions of cross-linked silicone particles and aqueous emulsions of oil containing cross-linked silicone particles were evaluated as described below.
- Average Particle Size of Crosslinked Silicone Particles in Aqueous Suspensions
   A median diameter (particle size corresponding to 50 % of the cumulative distribution: 50 % particle size) obtained by examining an aqueous suspension using a laser diffraction particle size analyzer (model LA-500, from Horiba, Ltd.) was used as the average particle size of the cross-linked silicone particles. Additionally, the particle size of 90 % was designated as the 90 % particle size.
- Average Particle Size of Oil Droplets in Aqueous Emulsions
   A median diameter (particle size corresponding to 50 % of cumulative distribution: 50 % particle size) obtained by examining an aqueous emulsion using a laser diffraction particle size analyzer (model LA-500, from Horiba, Ltd.) was used as the average particle size of oil droplets. Additionally, the particle size of 90 % was designated as the 90 % particle size.
- Average Particle Size of Crosslinked Silicone Particles in Aqueous Emulsions
   An oil composition containing cross-linked silicone particles obtained by removing water from an aqueous emulsion was examined under a microscope to obtain an average value for the particle size of ten cross-linked silicone particles.
- Stability
   After diluting an aqueous suspension or an aqueous emulsion with water to a solid matter concentration of 50 % by weight, 150 mL of it was placed in a 225-mL mayonnaise jar and left stand for two weeks at 50 °C. Stability was evaluated by measuring the thickness of the water layer that separated two weeks later. In addition, a smaller thickness indicates superior stability.

### [Practical Example 1]

94.8 parts by weight of a 400 mm²/s dimethylpolysiloxane having both ends of the molecular chain blocked by dimethylvinylsiloxy groups and containing 2.5 % by weight of an approximately 20 mm²/s cyclic dimethylsiloxane oligomer mixture were uniformly mixed with 5.2 parts by weight (enough to set the molar ratio of silicon-bonded hydrogen atoms to vinyl groups in the above-mentioned dimethylpolysiloxane to 0.95) of a 50 mm²/s copolymer of methylhydrogensiloxane and dimethylsiloxane having both ends of the molecular chain blocked by trimethylsiloxy groups (content of silicon-bonded hydrogen atoms = 0.31 % by weight).

Next, after adding an aqueous solution consisting of 0.5 parts by weight of sodium N-lauroyl-N-methyltaurine and 20 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1 ,3-divinyltetramethyldisiloxane complex of platinum in 1,3-divinyltetramethyldisiloxane and isopropyl alcohol with an aqueous solution consisting of 1 part by weight of ion exchange water and 0.01 parts by weight of the same ingredient as above, sodium N-lauroyl-N-methyltaurine.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous suspension of cross-linked silicone particles containing a mixture of cyclic dimethylsiloxane oligomers. The stability of the resultant aqueous suspension and the average particle size of the cross-linked silicone particles in it were measured. The results are shown in Table 1.

### [Practical Example 2]

94.8 parts by weight of a 400 mm²/s dimethylpolysiloxane having both ends of the molecular chain blocked by dimethylvinylsiloxy groups and containing 2.5 % by weight of an approximately 20 mm²/s cyclic dimethylsiloxane oligomer mixture were uniformly mixed with 5.2 parts by weight (enough to set the molar ratio of silicon-bonded hydrogen atoms to vinyl groups in the above-mentioned dimethylpolysiloxane to 0.95) of a 50 mm²/s copolymer of methylhydrogensiloxane and dimethylsiloxane having both ends of the molecular chain blocked by trimethylsiloxy groups (content of silicon-bonded hydrogen atoms = 0.31 % by weight).

Next, after adding an aqueous solution consisting of 0.5 parts by weight of sodium N-myristyl-N-methyltaurine and 20 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 1 part by weight of ion exchange water and 0.01 parts by weight of the same ingredient as above, sodium N-myristyl-N-methyltaurine.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous suspension of cross-linked silicone particles containing a mixture of cyclic dimethylsiloxane oligomers. The stability of the resultant aqueous suspension and the average particle size of the cross-linked silicone particles in it were measured. The results are shown in Table 1.

### [Comparative Example 1]

94.8 parts by weight of a 400 mm²/s dimethylpolysiloxane having both ends of the molecular chain blocked by dimethylvinylsiloxy groups and containing 2.5 % by weight of an approximately 20 mm²/s cyclic dimethylsiloxane oligomer mixture were uniformly mixed with 5.2 parts by weight (enough to set the molar ratio of silicon-bonded hydrogen atoms to vinyl groups in the above-mentioned dimethylpolysiloxane to 0.95) of a 50 mm²/s copolymer of methylhydrogensiloxane and dimethylsiloxane having both ends of the molecular chain blocked by trimethylsiloxy groups (content of silicon-bonded hydrogen atoms = 0.31 % by weight).

Next, after adding an aqueous solution consisting of 0.5 parts by weight of sodium lauryl sulfate and 20 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 1 part by weight of ion exchange water and 0.01 parts by weight of the same ingredient as above, sodium lauryl sulfate.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous suspension of cross-linked silicone particles containing a mixture of cyclic dimethylsiloxane oligomers. The stability of the resultant aqueous suspension and the average particle size of the cross-linked silicone particles in it were measured. The results are shown in Table 1.

### [Comparative Example 2]

94.8 parts by weight of a 400 mm²/s dimethylpolysiloxane having both ends of the molecular chain blocked by dimethylvinylsiloxy groups and containing 2.5 % by weight of an approximately 20 mm²/s cyclic dimethylsiloxane oligomer mixture were uniformly mixed with 5.2 parts by weight (enough to set the molar ratio of silicon-bonded hydrogen atoms to vinyl groups in the above-mentioned dimethylpolysiloxane to 0.95) of a 50 mm²/s copolymer of methylhydrogensiloxane and dimethylsiloxane having both ends of the molecular chain blocked by trimethylsiloxy groups (content of silicon-bonded hydrogen atoms = 0.31 % by weight).

Next, after adding an aqueous solution consisting of 2.0 parts by weight of a 25 % aqueous solution of sodium polyoxyethylene (2) lauryl sulfate and 28.5 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 0.6 parts by weight of ion exchange water and 0.04 parts by weight of the same ingredient as above, a 25 % aqueous solution of sodium polyoxyethylene (2) lauryl sulfate.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous suspension of cross-linked silicone particles containing a mixture of cyclic dimethylsiloxane oligomers. The stability of the resultant aqueous suspension and the average particle size of the cross-linked silicone particles in it were measured. The results are shown in Table 1.

**[Table 1]**

| | | Practical Example 1 | Practical Example 1 | Comparative Example 1 | Comparative Example 2 |
|---|---|---|---|---|---|
| Particle size of cross-linked silicone particles | | | | | |
| | Average particle size (µm) | 4.7 | 4.8 | 4.9 | 5.1 |
| | 90% particle size (µm) | 8.6 | 8.7 | 8.6 | 8.9 |
| Stability (mm) | | 5-8 | 5-8 | 5-8 | 5-8 |

### [Practical Example 3]

18.02 parts by weight of a 400 mm²/s copolymer of dimethylsiloxane and methylvinylsiloxane having both ends of the molecular chain blocked by dimethylvinylsiloxy groups (vinyl group content = 1.18 % by weight), 1.98 parts by weight of a 55 mm²/s copolymer of methylhydrogensiloxane and dimethylsiloxane (content of silicon-bonded hydrogen atoms = 0.48 % by weight) having both ends of the molecular chain blocked by trimethylsiloxy groups (in a quantity sufficient to bring the molar ratio of the silicon-bonded hydrogen atoms to the vinyl groups in the above-mentioned copolymer of methylvinylsiloxane and dimethylsiloxane to 0.95), and 80 parts by weight of a 100 mm²/s dimethylpolysiloxane having both ends of the molecular chain blocked by trimethylsiloxy groups were uniformly mixed.

Next, after adding an aqueous solution consisting of 0.5 parts by weight of sodium N-lauroyl-N-methyltaurine and 20 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 1 part by weight of ion exchange water and 0.01 parts by weight of the same ingredient as above, sodium N-lauroyl-N-methyltaurine.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous emulsion of dimethylpolysiloxane oil containing cross-linked silicone particles. The stability of the resultant aqueous emulsion, the average particle size of the cross-linked silicone particles, and the average particle size of the dimethylpolysiloxane oil droplets were measured. The results are shown in Table 2.

### [Practical Example 4]

18.02 parts by weight of a 400 mm²/s copolymer of dimethylsiloxane and methylvinylsiloxane having both ends of the molecular chain blocked by dimethylvinylsiloxy groups (vinyl group content = 1.18 % by weight), 1.98 parts by weight of a 55 mm²/s copolymer of methylhydrogensiloxane and dimethylsiloxane (content of silicon-bonded hydrogen atoms = 0.48 % by weight) having both ends of the molecular chain blocked by trimethylsiloxy groups (in a quantity sufficient to bring the molar ratio of the silicon-bonded hydrogen atoms to the vinyl groups in the above-mentioned copolymer of methylvinylsiloxane and dimethylsiloxane to 0.95), and 80 parts by weight of a 100 mm²/s dimethylpolysiloxane having both ends of the molecular chain blocked by trimethylsiloxy groups were uniformly mixed.

Next, after adding an aqueous solution consisting of 0.5 parts by weight of sodium N-myristyl-N-methyltaurine and 20 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 1 part by weight of ion exchange water and 0.01 parts by weight of the same ingredient as above, sodium N-myristyl-N-methyltaurine.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous emulsion of dimethylpolysiloxane oil containing cross-linked silicone particles. The stability of the resultant aqueous emulsion, the average particle size of the cross-linked silicone particles, and the average particle size of the dimethylpolysiloxane oil droplets were measured. The results are shown in Table 2.

### [Comparative Example 3]

18.02 parts by weight of a 400 mm²/s copolymer of dimethylsiloxane and methylvinylsiloxane having both ends of the molecular chain blocked by dimethylvinylsiloxy groups (vinyl group content = 1.18 % by weight), 1.98 parts by weight of a 55 mm²/s copolymer of methylhydrogensiloxane and dimethylsiloxane (content of silicon-bonded hydrogen atoms = 0.48 % by weight) having both ends of the molecular chain blocked by trimethylsiloxy groups (in a quantity sufficient to bring the molar ratio of the silicon-bonded hydrogen atoms to the vinyl groups in the above-mentioned copolymer of methylvinylsiloxane and dimethylsiloxane to 0.95), and 80 parts by weight of a 100 mm²/s dimethylpolysiloxane having both ends of the molecular chain blocked by trimethylsiloxy groups were uniformly mixed.

Next, after adding an aqueous solution consisting of 0.5 parts by weight of sodium lauryl sulfate and 20 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 1 part by weight of ion exchange water and 0.01 parts by weight of the same ingredient as above, sodium lauryl sulfate.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous emulsion of dimethylpolysiloxane oil containing cross-linked silicone particles. The stability of the resultant aqueous emulsion, the average particle size of the cross-linked silicone particles, and the average particle size of the dimethylpolysiloxane oil droplets were measured. The results are shown in Table 2.

### [Comparative Example 4]

18.02 parts by weight of a 400 mm²/s copolymer of dimethylsiloxane and methylvinylsiloxane having both ends of the molecular chain blocked by dimethylvinylsiloxy groups (vinyl group content = 1.18 % by weight), 1.98 parts by weight of a 55 mm²/s copolymer of methylhydrogensiloxane and dimethylsiloxane (content of silicon-bonded hydrogen atoms = 0.48 % by weight) having both ends of the molecular chain blocked by trimethylsiloxy groups (in a quantity sufficient to bring the molar ratio of the silicon-bonded hydrogen atoms to the vinyl groups in the above-mentioned copolymer of methylvinylsiloxane and dimethylsiloxane to 0.95), and 80 parts by weight of a 100 mm²/s dimethylpolysiloxane having both ends of the molecular chain blocked by trimethylsiloxy groups were uniformly mixed. Next, after adding an aqueous solution consisting of 2.0 parts by weight of a 25 % aqueous solution of sodium polyoxyethylene (2) lauryl sulfate and 28.5 parts by weight of pure water and emulsifying the mixture in a colloid mill, an aqueous emulsion of a silicone composition was prepared by diluting the mixture with 39 parts by weight of pure water.

Separately, an aqueous emulsion of a platinum catalyst with an average particle size of 0.3 µm was prepared by uniformly mixing 1 part by weight of a mixed solution of 1,3-divinyltetramethyldisiloxane complex of platinum in 1,3-divinyltetramethyldisiloxane and isopropyl alcohol with an aqueous solution consisting of 0.6 parts by weight of ion exchange water and 0.04 parts by weight of the same ingredient as above, a 25% aqueous solution of sodium polyoxyethylene (2) lauryl sulfate.

Next, after adding the above-mentioned aqueous emulsion of a platinum catalyst (in a quantity sufficient to bring the amount of platinum metal in the silicone composition in weight units to 5 ppm) to the above-mentioned aqueous emulsion of a silicone composition and uniformly mixing them, the mixture was left stand for one day so as to crosslink the silicone composition by means of a hydrosilylation reaction, yielding an aqueous emulsion of dimethylpolysiloxane oil containing cross-linked silicone particles. The stability of the resultant aqueous emulsion, the average particle size of the cross-linked silicone particles, and the average particle size of the dimethylpolysiloxane oil droplets were measured. The results are shown in Table 2.

**[Table 2]**

| | | Practical Example 3 | Practical Example 4 | Comparative Example 3 | Comparative Example 4 |
|---|---|---|---|---|---|
| Particle size of dimethylpolysiloxane oil droplets | | | | | |
| | Average particle size (µm) | 4.0 | 4.3 | 4.3 | 4.5 |
| | 90% particle size (µm) | 7.4 | 8.4 | 7.5 | 8.0 |
| Particle size of cross-linked silicone particles | | | | | |
| | Average particle size (µm) | 3 | 3 | 3 | 3 |
| Stability (mm) | | 4-7 | 4-7 | 4-7 | 4-7 |

### [Practical Example 5]

Skin care shampoo compositions (1) and (2), whose formulations are listed in Table 3, were prepared using the aqueous suspension of cross-linked silicone particles prepared in Practical Example 1 or the aqueous emulsion of dimethylpolysiloxane oil containing cross-linked silicone particles prepared in Practical Example 4. The sensory properties and stability in mixtures of the skin care shampoo compositions were evaluated in accordance with the following methods. The results are shown in Table 3.

### • Stability in mixtures

Stability was evaluated by the appearance of the shampoo composition immediately after preparation and after one month's storage at 50 °C. The evaluation results were tabulated in the following manner.
⊚: The composition was uniform, with no changes noted.
Δ: Slight creaming noted in the upper portion.
×: Completely separated into two layers.

### • Sensory Properties

The skin care shampoo compositions were used to cleanse the forearms of 5 panelists for 30 seconds, after which compositions were washed off under running water. Next, their sensory properties were evaluated after wiping off the moisture with a dry towel and drying the skin.

### [Comparative Example 5]

Skin care shampoo compositions (3) and (4), whose formulations are listed in Table 3, were prepared using the aqueous suspension of cross-linked silicone particles prepared in Comparative Example 1 or the aqueous emulsion of dimethylpolysiloxane oil containing cross-linked silicone particles prepared in Comparative Example 4. The sensory properties and stability in mixtures of the skin care shampoo compositions were evaluated in the same manner as in Practical Example 5. The results are shown in Table 3.

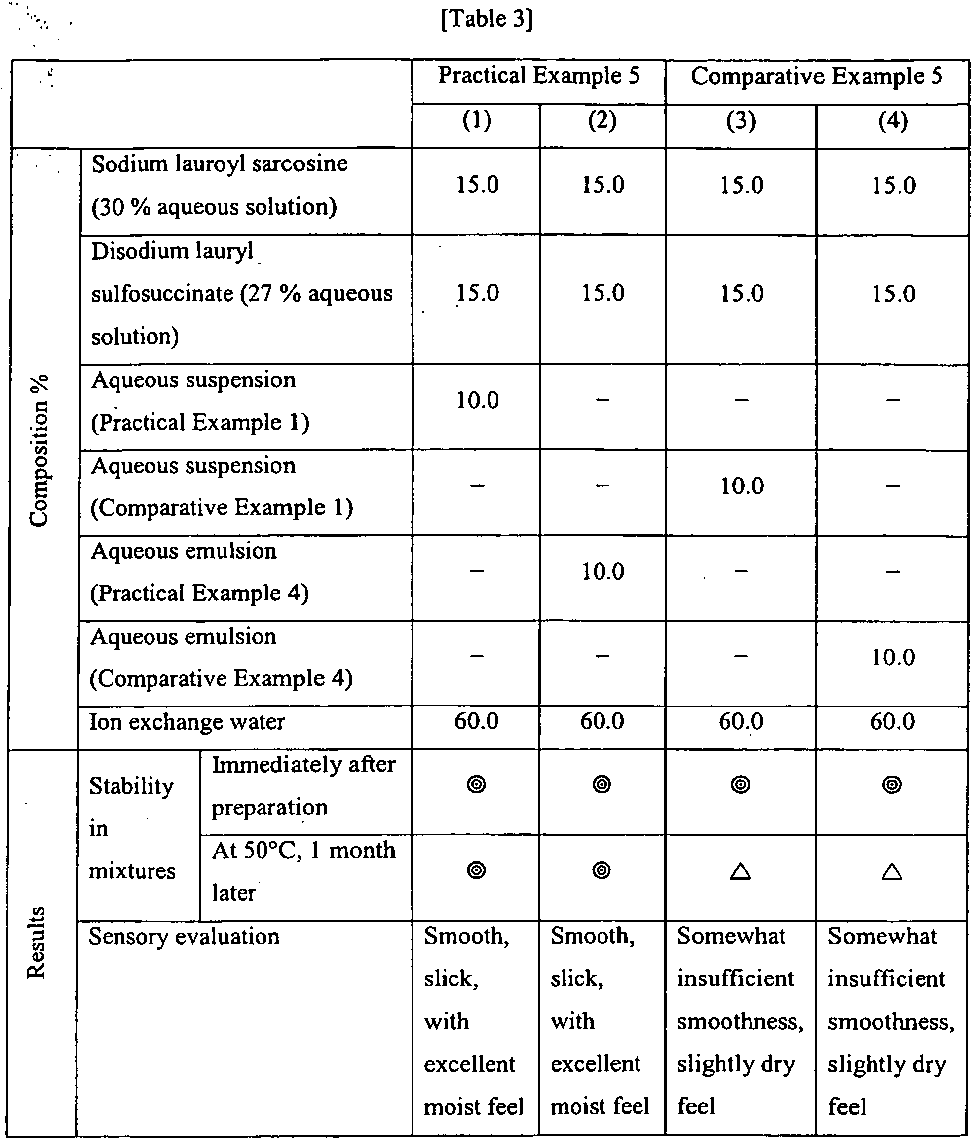

### Industrial Applicability

The inventive aqueous suspension of cross-linked silicone particles and aqueous emulsion of oil containing cross-linked silicone particles are characterized by superior stability and by low environmental impact and minimal effects on the human body due to the use of special N-acyl-, N-hydrocarbon taurines or their salts as surface active agents. Such inventive aqueous suspensions and aqueous emulsions are extremely useful as cosmetic raw materials because of their superior compatibility with the human body and superior functionality such as wetness and smoothness.

## Claims

1. Aqueous suspensions of cross-linked silicone particles comprising :
(A) cross-linked silicone particles with an average particle size of from 0.5 to 500 µm,
(B) N-acyl-, N-hydrocarbon taurines represented by the general formula (I); 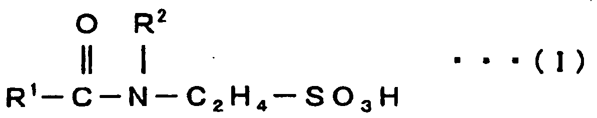 (where R¹ and R² stand for unsubstituted or substituted monovalent hydrocarbon groups) and/or their salts, and
(C) water.

2. The aqueous suspensions according to claim 1, wherein component (A) consists of cross-linked silicone particles containing non-crosslinkable oil.

3. The aqueous suspensions according to claim 1, wherein component (A) accounts for from 25 to 80 % by weight, component (B) accounts for from 0.001 to 20 % by weight, and component (C) accounts for from 5 to 75 % by weight.

4. Aqueous emulsions of oil containing cross-linked silicone particles comprising :
(A) cross-linked silicone particles with an average particle size of from 0.5 to 500 µm,
(D) oil,
(B) N-acyl-, N-hydrocarbon taurines represented by the general formula (I) 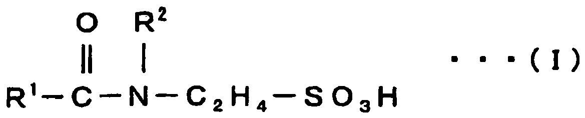 (where R¹ and R² stand for unsubstituted or substituted monovalent hydrocarbon groups) and/or their salts, and
(C) water,
with component (A) contained in droplets of component (D) dispersed in water.

5. The aqueous emulsions according to claim 4, wherein component (D), which contains component (A) accounts for from 25 to 90 % by weight, component (B) accounts for from 0.001 to 20 % by weight, and component (C) accounts for from 5 to 75 % by weight.

6. Cosmetic raw materials comprising the aqueous suspensions according to any one of claims 1 through 3.

7. Cosmetic raw materials comprising the aqueous emulsions according to claim 4 or claim 5.

## Patentansprüche

1. Wässrige Suspensionen von vernetzten Silikonpartikeln, die Folgendes umfassen:
(A) vernetzte Silikonpartikel mit einer durchschnittlichen Partikelgröße von 0,5 bis 500 µm,
(B) N-Acyl-, N-Kohlenwasserstofftaurine, die durch die allgemeine Formel (I) dargestellt sind: 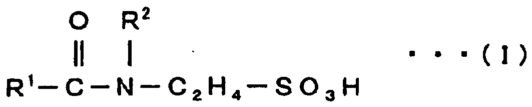 (wo R¹ und R² unsubstituierte oder substituierte monovalente Kohlenwasserstoffgruppen repräsentieren)
und/oder ihre Salze, und
(C) Wasser.

2. Wässrige Suspensionen nach Anspruch 1, worin Komponente (A) aus vernetzten Silikonpartikeln besteht, die nichtvernetzbares Öl enthalten.

3. Wässrige Suspensionen nach Anspruch 1, worin Komponente (A) von 25 bis 80 Gewichts-% beträgt, Komponente (B) von 0,001 bis 20 Gewichts-% beträgt und Komponente (C) von 5 bis 75 Gewichts-% beträgt.

4. Wässrige, vernetzte Silikonpartikel enthaltende Emulsionen von Öl, die Folgendes umfassen:
(A) vernetzte Silikonpartikel mit einer durchschnittlichen Partikelgröße von 0,5 bis 500 µm,
(D) Öl,
(B) N-Acyl-, N-Kohlenwasserstofftaurine, die durch die allgemeine Formel (I) dargestellt sind: 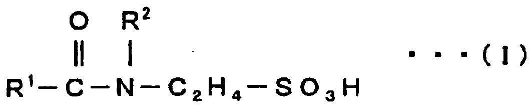 (wo R¹ und R² unsubstituierte oder substituierte monovalente Kohlenwasserstoffgruppen repräsentieren)
und/oder ihre Salze, und
(C) Wasser,
wobei Komponente (A) in Tröpfchen der Komponente (D) enthalten ist, die in Wasser dispergiert sind.

5. Wässrige Emulsionen nach Anspruch 4, worin Komponente (D), die Komponente (A) enthält, von 25 bis 90 Gewichts-% beträgt, Komponente (B) von 0,001 bis 20 Gewichts-% beträgt und Komponente (C) von 5 bis 75 Gewichts-% beträgt.

6. Kosmetische Rohstoffe, die die wässrigen Suspensionen nach einem der Ansprüche 1 bis 3 umfassen.

7. Kosmetische Rohstoffe, die die wässrigen Emulsionen nach Anspruch 4 oder Anspruch 5 umfassen.

## Revendications

1. Suspensions aqueuses de particules de silicone réticulées comprenant :
(A) des particules de silicone réticulées avec une taille de particule moyenne allant de 0,5 à 500 µm,
(B) des taurines N-acyle, N-hydrocarbure représentées par la formule générale (I), 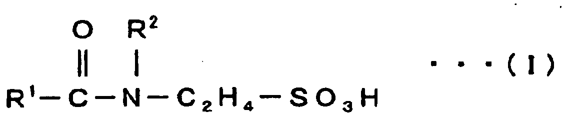 (où R¹ et R² sont des groupes hydrocarbures monovalents non substitués ou substitués)
et/ou leurs sels, et
(C) de l'eau.

2. Suspensions aqueuses selon la revendication 1, dans lesquelles le composant (A) consiste en des particules de silicone réticulées contenant une huile non réticulable.

3. Suspensions aqueuses selon la revendication 1, dans lesquelles le composant (A) représente de 25 à 80 % en poids, le composant (B) représente de 0,001 à 20 % en poids, et le composant (C) représente de 5 à 75 % en poids.

4. Emulsions aqueuses d'huile comprenant des particules de silicone réticulées comprenant :
(A) des particules de silicone réticulées avec une taille de particule moyenne allant de 0,5 à 500 µm,
(D) de l'huile,
(B) des taurines N-acyle, N-hydrocarbure représentées par la formule générale (I) 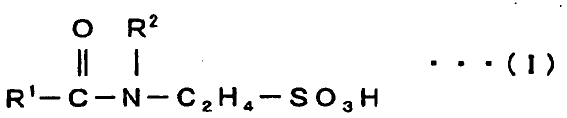 (où R¹ et R² sont des groupes hydrocarbures monovalents non substitués ou substitués)
et/ou leurs sels, et
(C) de l'eau,
avec le composant (A) contenu dans des gouttelettes de composant (D) dispersé dans de l'eau.

5. Emulsions aqueuses selon la revendication 4, dans lesquelles le composant (D), qui contient le composant (A) représente de 25 à 90 % en poids, le composant (B) représente de 0,001 à 20 % en poids, et le composant (C) représente de 5 à 75 % en poids.

6. Matières premières cosmétiques comprenant les suspensions aqueuses selon l'une quelconque des revendications 1 à 3.

7. Matières premières cosmétiques comprenant les émulsions aqueuses selon la revendication 4 ou la revendication 5.
